# EUROPEAN PATENT SPECIFICATION

(11) **EP 4 025 154 B1**
(45) Date of publication and mention of the grant of the patent: **11.11.2026**
(21) Application number: 20859815.1
(22) Date of filing: 04.09.2020
(51) Int. Cl.: A61C 5/77, A61B 6/03, A61C 19/04, A61C 3/02, A61C 5/70

(54) **A COMPUTER-IMPLEMENTED METHOD FOR GENERATING A PREPARATION SURFACE OF A TOOTH OF A SUBJECT**
COMPUTERIMPLEMENTIERTES VERFAHREN ZUR ERZEUGUNG EINER PRÄPARATIONSFLÄCHE EINES ZAHNS EINES PATIENTEN
PROCÉDÉ MIS EN OEUVRE PAR ORDINATEUR POUR GÉNÉRER UNE SURFACE DE PRÉPARATION D'UNE DENT D'UN SUJET

(30) Priority: 06.09.2019 US 201962896885 P; 14.11.2019 US 201962935261 P
(43) Date of publication of application: 13.07.2022
(73) Proprietor: Cyberdontics (USA), Inc., San Francisco, CA 94123 (US)
(72) Inventor: CIRIELLO, Christopher, John, San Francisco, CA 94123 (US); JACKSON, James, San Francisco, CA 94123 (US); MULLER, Nathan, John, San Francisco, CA 94123 (US); PARKER, Niall, San Francisco, CA 94123 (US)
(74) Representative: Lucke, Andreas
(86) International application number: PCT/IB2020/000729
(87) International publication number: WO 2021/044218

(56) References cited:
- WO-A1-2018/154485
- WO-A1-2018/154485
- DE-A1- 10 145 104
- DE-A1- 10 145 104
- US-A1- 2006 127 848
- US-A1- 2015 320 320

## Description

### CROSS REFERENCE

### BACKGROUND

The goal of a crown restoration is to replace damaged, missing or decayed tooth structure with a suitable restorative material (the crown) while retaining as much of the sound natural tooth as possible. The interface between crown and tooth consists of the intaglio surface of the crown, a cement gap and the tooth preparation.

With regard to the prior art, reference is directed to WO2021044218A1, which discloses an automated drill system that includes dental tooth scanner for creating an image of the subject's tooth. A control program creates a 3D image from the data received from dental tooth scanner. In a variation, central processing unit renders a three-dimensional model of the target tooth using a deformable pre-surgical mesh with a bitewing X-ray overlaid. The deformable pre-surgical mesh is editable by the user to produce a postsurgical mesh and a representation of the target restoration (restoration mesh). The three-dimensional model presents a rendering of surgical criteria to the user regarding treatment. The tool path for the automated drill is parametrically generated from a view port with a bitewing or periapical radiograph registered to the 3d mesh. This allows the clinical to avoid anatomical landmarks such as the pulp, adjacent teeth, or remove caries. The tool path is parametrically generated with 6 user inputs: tooth number being operated on, the restoration material which determines the material thickness required, the opposing dental arch mesh, and 3 clicks. The three clicks are (1 - margin placement), 2 margin widths, and 3 preparation wall taper and occlusal reduction.

Reference is further directed to DE 101 45 104 A1, which discloses a method for producing dental crowns or dental bridges from a three-dimensionally measured and digitized model of tooth stumps that have been prepared by a dentist, in a conventional manner. The document teaches that a predetermined offset value should be applied to the inner surface of the model of the crown or bridge, so as to form a cement gap between the crown or bridge, and the tooth stump. Further prior art is exemplified by WO 2018/154485 A1.

### SUMMARY OF THE INVENTION

The present invention is as defined by the independent claims 1, 11 and 12. Additional features and embodiments of the invention are defined in the dependent claims. In one aspect, disclosed herein is a method for generating a preparation surface of a tooth of a subject, the method comprising: a. receiving surface data of the tooth of the subject; b. determining one or more parameters of the tooth of the subject by analyzing the surface data; and c. generating the preparation surface using the one or more parameters; d. wherein the preparation surface of the tooth comprises a three-dimensional model of the surface of the tooth, an intended cut region, or both. In some embodiments, the surface data comprise two-dimensional X-ray images of the tooth. In some embodiments, the two-dimensional X-ray images of the tooth are taken along at least two planes that are not parallel. In some embodiments, the surface data comprises two-dimensional Computed Tomography (CT) images of the tooth. In some embodiments, the surface data comprises three-dimensional images of the tooth. In some embodiments, the surface data comprises images of the tooth and other teeth of the subject. In some embodiments, the one or more parameters comprise one or more of: a top surface of the tooth, an edge of the tooth, an envelope of the tooth, a central axis of the tooth. In some embodiments, the edge or envelope of the tooth is three-dimensional. In some embodiments, the edge connects a top surface of the tooth. In some embodiments, the method further comprises, prior to b, determining an extent of tooth decay by analyzing the surface data. In some embodiments, the method further comprises determining a top surface of the preparation surface based on the extent of tooth decay. In some embodiments, the method further comprises determining an edge of the preparation surface based on the extent of tooth decay. In some embodiments, the method further comprises selecting a marginal finish type, one or more draft angles of the preparation surface, or both, prior to c. In some embodiments, the method further comprises, prior to c, determining a shape of a top surface of the preparation surface. In some embodiments, the method further comprises, subsequent to c, generating a crown cavity surface automatically by adding a pre-determined gap space to the preparation surface. In some embodiments, the pre-determined gap space is based on manufacturing tolerance of a crown cavity, a machining tolerance of the crown preparation surface, a desired marginal gap, or any combination thereof. In some embodiments, machining of the crown preparation surface is by a system configured for a dental procedure, and wherein the system configured for the dental procedure 1) is an automated dental drill (ADD) system configured for tooth cutting or tooth drilling; and/or 2) comprises a laser generating source. In some embodiments, the method further comprises, subsequent to c, transmitting the crown preparation surface to a system configured for toolpath generation or machining of the tooth. In some embodiments, the method further comprises, subsequent to c, transmitting the crown preparation surface to a system configured for a dental procedure. In some embodiments, the system configured for the dental procedure 1) is an automated dental drill (ADD) system configured for tooth cutting or tooth drilling; and/or 2) comprises a laser generating source. In some embodiments, the surface data of the tooth is generated with occlusion by a tooth adjacent thereof or a gum. In some embodiments, the surface data of the tooth is generated without occlusion of the tooth by a tooth adjacent thereof or a gum. In some embodiments, the surface data of the tooth is generated when additional space is created between the tooth and an adjacent tooth or between the tooth and a gum via insertion of a dental wedge, a retraction cord, a string, or a combination thereof. In some embodiments, the tooth is not occluded by the adjacent tooth or the gum. In some embodiments, the method further comprises, subsequent to a and prior to b, processing the surface data. In some embodiments, processing the surface data comprises using interpolation for estimating interproximal contact, occluded interproximal contact, occluded subgingival contact, or any combination thereof. In some embodiments, processing the surface data comprises segmenting the surface data into one or more groups, wherein at least one group represents of the tooth of the subject. In some embodiments, processing the surface data comprises progressively intersecting a plane along the x-y direction with the surface data to determine a width, a nominal center, or both of the teeth. In some embodiments, the method further comprises prior to a), inserting a separator between the tooth and an adjacent tooth thereof, the separator comprising one or more fiducial markers thereon; generating the surface data of the tooth with the one or more fiducial markers; and estimating the interproximal contact using the surface data with the one or more fiducial markers. In some embodiments, the separator is a thin strip. In some embodiments, an error in the estimated interproximal contact is less than 20 µm.

In some embodiments, generating the one or more missing surface patches is performed by a machine learning algorithm. In some embodiments, the one or more missing surface patches comprise an occluded region between teeth, an interproximal region between teeth, a subgingival tooth surface, or any combination thereof. In some embodiments, the one or more missing surface patches are generated by interpolation of an expected surface from existing scanned geometries. In some embodiments, the one or more missing surface patches are generated by a machine learning algorithm, a neural network, or any combination thereof. In some embodiments,the one or more missing surface patches are generated by normalized tooth geometries from marked samples. In some embodiments, the one or more missing surface patches are generated by combining conventional dental scanning with optical coherence tomography of occluded or hidden (subgingival) surfaces. In some embodiments, the method further comprises generating a prosthetic external surface based on a volumetric boundary for the prosthetic. In some embodiments, the prosthetic external surface includes a proximal contact of an adjacent tooth. In some embodiments, the method further comprises generating an internal surface of the prosthetic based at least on the prosthetic external surface. In some embodiments, the method further comprises performing an iterative Finite Element Analysis (FEA) to optimize the shape of prosthetic internal surface for reduced stress forces. In some embodiments, the method further comprises generating a crown preparation surface based on the surface data of the tooth, the one or more parameters of the tooth, the preparation surface, the three-dimensional model of the surface of the tooth, the cut region, or any combination thereof.

In another aspect, disclosed herein is A method for cutting prosthetic preparation margins of a tooth, the method comprising: a. receiving diagnostic data of the tooth and a clinical parameter of the tooth; b. obtaining a geometrical shape of the prosthetic preparation margins of the tooth; c. selecting a method of material removal; d. using the selected method to automatically cut the tooth thereby generating the prosthetic preparation margins with the geometrical shape. In some embodiments, the diagnostic data comprises one or more of: observation data, surface mapping data, radiographic data, ultrasound data, or any combination thereof of the tooth, a tissue surrounding the tooth, or both. In some embodiments, the geometrical shape comprises one or more of: a chamfer, a knife edge, a radial shape, a radial shape with bevel, and a square. In some embodiments, the tooth is automatically cut with a cutting bit, a cutting bur, laser ablation, a water jet, an air jet, an abrasive, or any combination thereof. In some embodiments, d comprises using the selected method by a system configured for a dental procedure. In some embodiments, the system configured for the dental procedure 1) is an automated dental drill (ADD) system configured for tooth cutting or tooth drilling; and/or 2) comprises a laser generating source. In some embodiments, the method comprising: selecting one or more methods of material removal; and applying the one or more methods to perform circumferential and occlusion reductions thereby obtaining 1) a substantially consistent taper; 2) a substantially consistent reduction, or both. In some embodiments, the one or more methods comprise using a rotary stage to position a burr to a pre-determined taper. In some embodiments, the one or more methods comprise using a pre-determined taper on a bur. In some embodiments, the circumferential and occlusion reductions are configured to provide equal thickness or gap to a prosthetic crown to the tooth. In some embodiments, the circumferential and occlusion reductions are generated via angled side-wall cuts. In some embodiments, the circumferential and occlusion reductions are generated by a system configured for a dental procedure. In some embodiments, the system configured for a dental procedure 1) is an automated dental drill (ADD) system configured for tooth cutting or tooth drilling; and/or 2) comprises a laser generating source. In some embodiments, the laser generating source is configured to generate a laser beam with a wave length in the range of 5 µm to 15 µm. In some embodiments, the laser generating source is at or near a distal end of the system configured for the dental procedure. In some embodiments, the laser generating source is at a headpiece.

### BRIEF DESCRIPTION OF THE DRAWINGS

A better understanding of the features and advantages of the present subject matter will be obtained by reference to the following detailed description that sets forth illustrative embodiments and the accompanying drawings of which:
Fig. 1 shows a non-limiting exemplary two-dimensional (2D) X-ray image of the teeth of a subject including a decay, per one or more embodiments herein;
Fig. 2 shows a non-limiting exemplary the three-dimensional (3D) surface data of a tooth of a subject, per one or more embodiments herein;
Fig. 3 shows a non-limiting exemplary embodiment of linear translation of a plane along a central axis of the teeth and intersecting the 3D surface data of the teeth, per one or more embodiments herein;
Fig. 4 shows a non-limiting exemplary embodiment of progressive formation of a teeth surface as shown in Fig. 5A from intersecting the 3D surface data in Fig. 3, per one or more embodiments herein;
Fig. 5A shows a non-limiting exemplary embodiment of a 3D surface data of a tooth, per one or more embodiments herein;
Fig. 5B shows a non-limiting exemplary embodiment of a 3D crown preparation surface of a tooth, per one or more embodiments herein;
Fig. 6 shows a non-limiting exemplary embodiment of the prosthetic crown, crown preparation surface and crown cavity (intaglio) surface of a tooth, per one or more embodiments herein;
Fig. 7 shows a first non-limiting exemplary embodiment of a tooth separation element, per one or more embodiments herein in this case, per one or more embodiments herein a dental wedge, per one or more embodiments herein;
Fig. 8 shows a second non-limiting exemplary embodiment of a tooth separation element, per one or more embodiments herein in this case, per one or more embodiments herein a dental wedge, per one or more embodiments herein;
Fig. 9A shows a non-limiting exemplary embodiment of an over extended margin between a prosthetic crown and a tooth, per one or more embodiments herein;
Fig. 9B shows a non-limiting exemplary embodiment of an under extended margin between a prosthetic crown and a tooth, per one or more embodiments herein;
Fig. 10 shows a non-limiting exemplary embodiment of reductions for an incisor tooth for installation of the prosthetic crown, per one or more embodiments herein ;
Fig. 11 shows a non-limiting perspective illustration of reductions for a molar or premolar tooth for installation prosthetic crown, per one or more embodiments herein;
Fig. 12 shows a non-limiting schematic diagram of a digital processing device; in this case, a device with one or more CPUs, a memory, a communication interface, and a display;
Fig. 13 shows a non-limiting schematic diagram of a web/mobile application provision system; in this case, a system providing browser-based and/or native mobile user interfaces; and
Fig. 14 shows a non-limiting schematic diagram of a cloud-based web/mobile application provision system; in this case, a system comprising an elastically load balanced, auto-scaling web server and application server resources as well synchronously replicated databases;
Figure 15 shows an image of an exemplary Cone-Beam Computed Tomography (DCCT), per one or more embodiments herein;

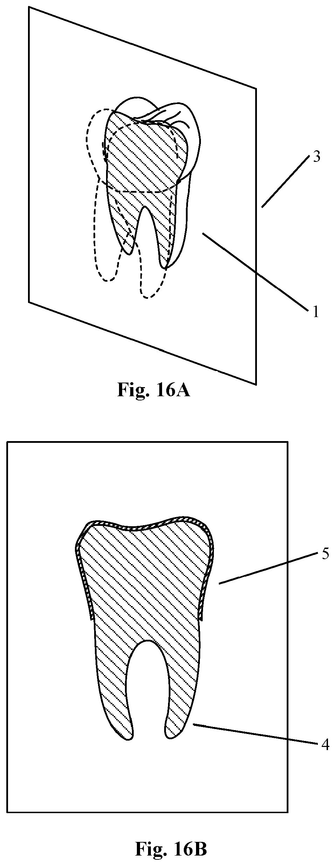

Figure 16A shows an image of an exemplary bitewing radiograph registered to a 3d surface geometry, or in another embodiment a 3d bitewing radiograph, per one or more embodiments herein;
Figure 16B shows an image of an exemplary 2D bitewing radiograph, per one or more embodiments herein;
Figure 17A shows an exemplary front-view illustration of burr forces, per one or more embodiments herein;
Figure 17B shows an exemplary top cross-sectioned view illustration of burr forces, per one or more embodiments herein; and
Figure 17C shows an exemplary detailed top cross-sectioned view illustration of burr forces, per one or more embodiments herein.

### DETAILED DESCRIPTION OF THE INVENTION

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. The scope of the invention is defined by the appended claims.

### Certain terms

Unless otherwise defined, all technical terms used herein have the same meaning as commonly understood by one of ordinary skill in the art to which this invention belongs.

As used herein, the singular forms "a," "an," and "the" include plural references unless the context clearly dictates otherwise. Any reference to "or" herein is intended to encompass "and/or" unless otherwise stated.

As used herein, the term "about" refers to an amount that is near the stated amount by 10%, 5%, or 1%, including increments therein.

As used herein, the term "about" in reference to a percentage refers to an amount that is greater or less the stated percentage by 10%, 5%, or 1%, including increments therein.

As used herein, the phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

### Crown preparation surface

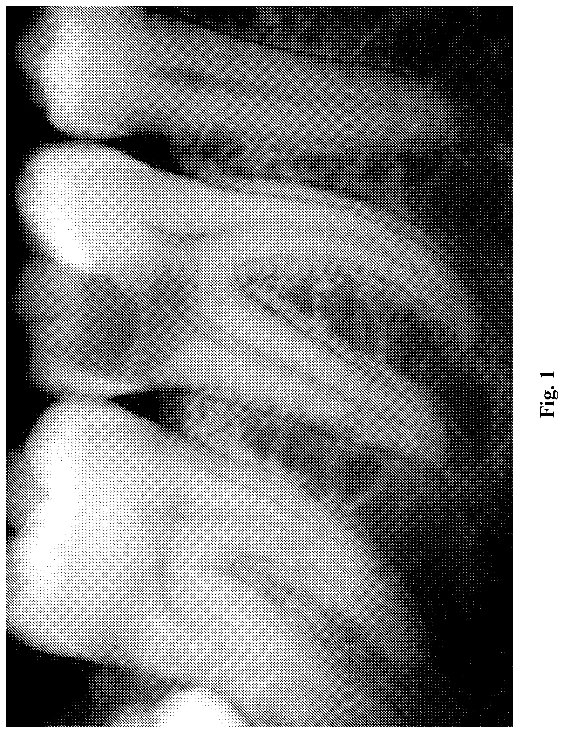

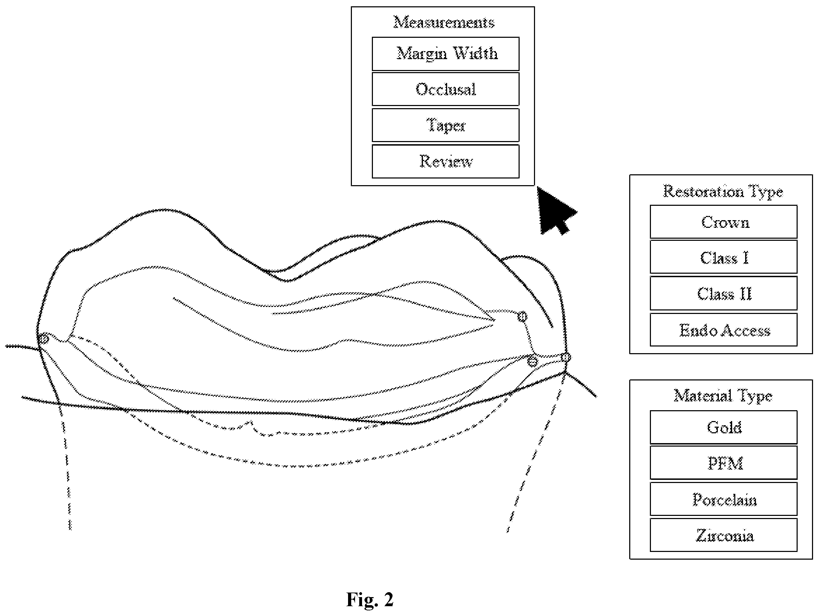

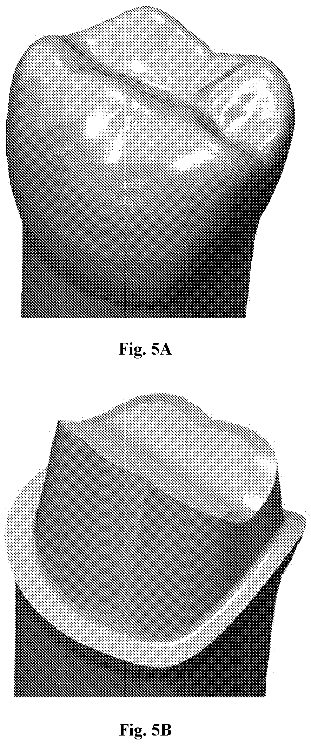

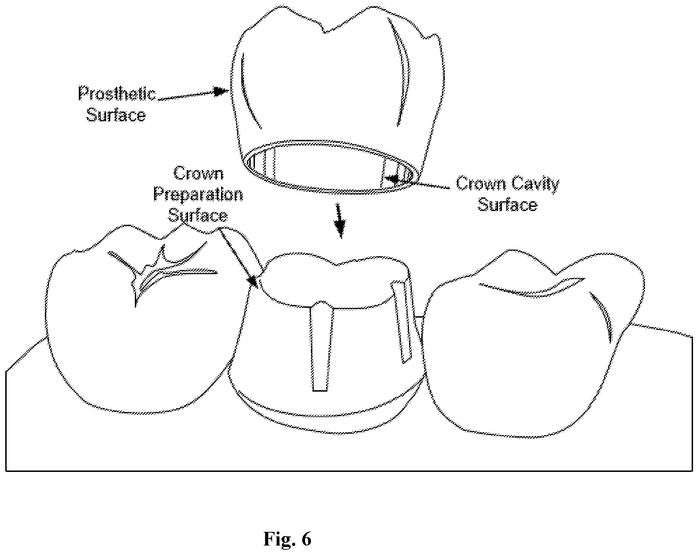

In some embodiments, disclosed herein are systems and methods herein that determine a dental crown preparation surface. In some embodiments, the systems and /or methods disclosed herein are computer-implemented. An exemplary embodiment of the crown preparation surface is shown in Fig. 5B and Fig. 6. In some embodiments, the systems and methods begin with radiological surface data. For example, 2D X-ray data as shown in Fig. 1, 2D CT data, or 3D images of the teeth. In some embodiments, the surface data include data with multiple orientations or data with full 3D details (e.g. Cone beam computed tomography (CBCT)). In some embodiments, the nominal center (e.g., center along z direction) of the tooth is selected by clinician. In some embodiments, the nominal center is selected automatically by a computer program.

After a nominal center is determined, image processing can then identify the tooth edge and determine the overall envelope and central axis of the subject tooth. An exemplary embodiment of the tooth edge(s), envelope, and/or central axis are shown in Fig. 2.

In some embodiments, the extent of decay on the top surface is determined based on density and sets the upper preparation plane while decay on the edges sets the lower preparation plane. An example of decay is shown in Fig. 1. Marginal finish types and draft angles of the crown preparation can be selected by the clinician or by a computer program automatically. Selection of the crown preparation and top surface geometry (e.g., circular, elliptical, polygon, or any other shapes) can be directed by the clinician, default to a standard shape, or by a computer program automatically. The output of this process can be a candidate preparation surface, representing a digital surface model of the tooth.

In some embodiments, the 3D surface model is located in a reference frame with an origin based on the adjacent teeth. This can provide the reference to accurately machine the tooth, for example, when the automated dental drill (ADD) is clamped to the custom mount. Inputs from imaging devices that capture snapshots of their field of view can be leveraged by being stitched together, forming a full 3D surface of the region of interest.

In some embodiments, given the candidate preparation surface, the crown cavity surface, as shown in Fig. 6, can be automatically determined by adding the required gap space to the preparation surface (growing outward based on the surface element normal axes). Minimum gap space for the preceding calculation can be based on manufacturing tolerance of a crown cavity, a machining tolerance of the crown preparation surface, a desired marginal gap, or any combination thereof. In some embodiments, the minimum gap space in the in the range of about 1nm to 10 mm. Combining the crown cavity surface with the outer crown surface (based on original tooth scans after corrections for wear and decay) results in a 3D volume of the manufactured crown. In some embodiments, this model is checked for minimum thicknesses (depending on the material chosen) and any areas where the material is too thin may require growing crown thickness. If the outer surface of the crown does not contact adjacent or opposing teeth then growing outward can save removal of the tooth, though this may not be desirable esthetically. In some embodiments, thin crown material requires growing the crown cavity surface inward, with the crown preparation surface moving inward accordingly to retain the required gap space.

In some embodiments, the final crown preparation surface after accommodation for crown thickness is presented for review to clinician. The crown cavity surface can be defined from the approved final crown preparation surface and provided for manufacture of the crown while the approved crown preparation surface is passed onto the ADD for toolpath generation (based on cutting tool parameters) and machine of the tooth.

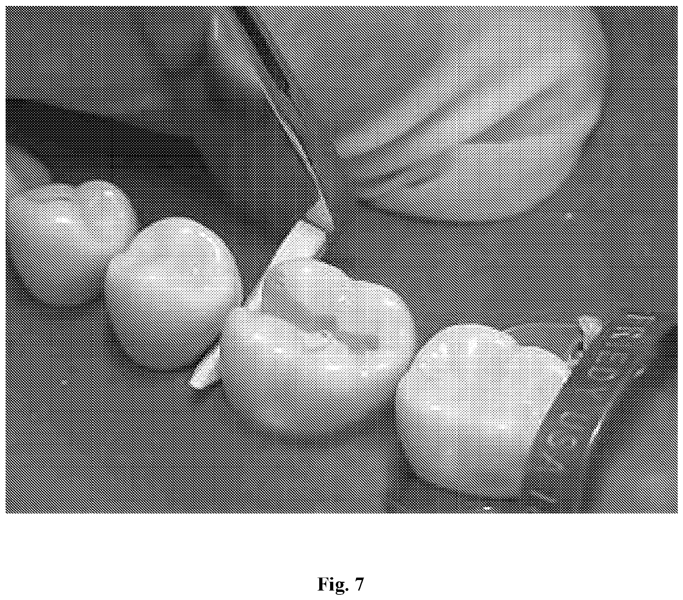

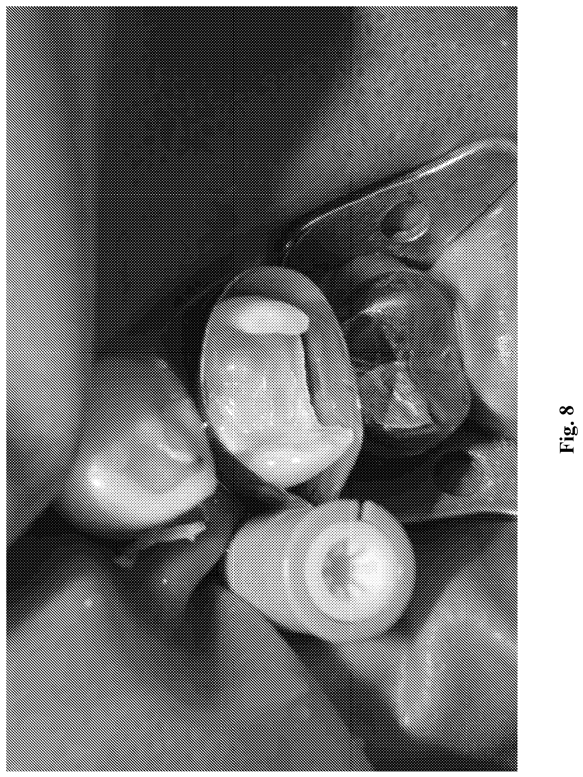

In some embodiments, 3D scanning of the tooth, e.g., X-ray or CT data, provides the initial models for the design of the crown and to define the crown preparation surface. The space between the teeth can be obscured resulting in an incomplete or joined surface model. Methods and apparatuses to complete and separate the subject and adjacent tooth models can be useful, for example, to aid automated intraoral cutting. With an initial surface scan and tooth position acting as a datum, a second surface scan of the teeth can occur through the separation of occluded teeth to provide visual access for scanning. In some embodiments, the separation of occluded teeth can be accomplished with a wedge for two adjacent teeth, e.g. in Figs. 7-8, or a string to recede the gums, depending on the specific type(s) of occlusion. In some embodiments, a datum can be an origin by which a common (Cartesian, cylindrical, spherical, etc.) coordinate system is set.

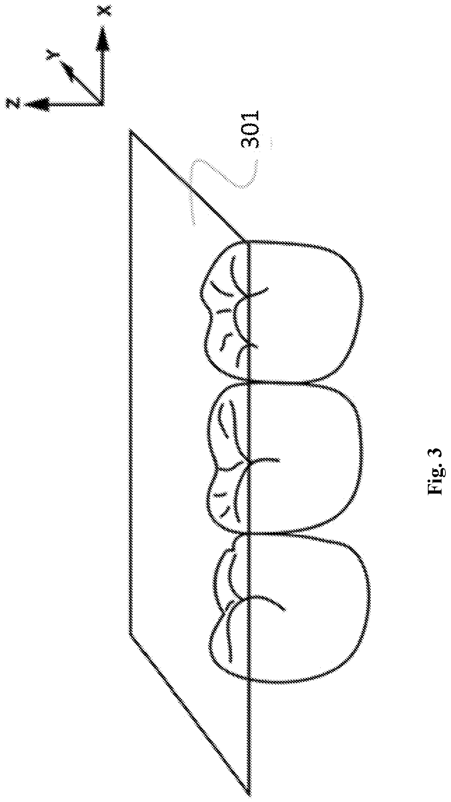

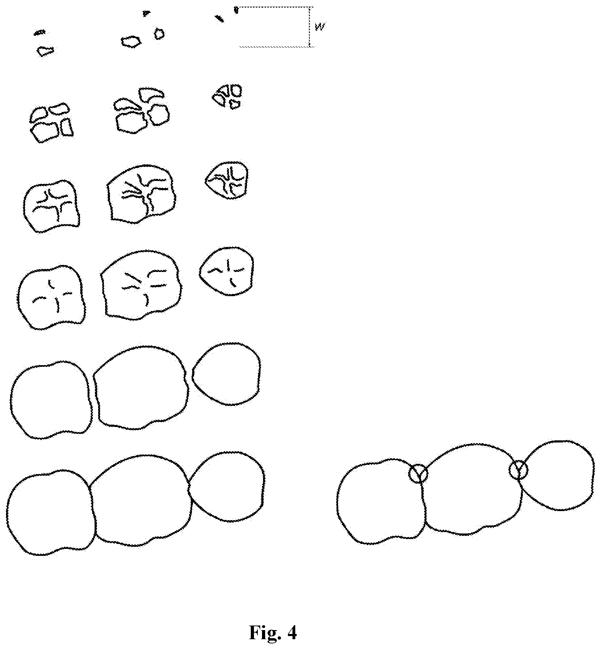

Given a raw 3D scan of a set of teeth, for example, as shown in Fig. 3, knowledge of typical tooth size and shape can be used to fill in the missing surface data. In some embodiments, the method herein includes progressively intersecting a plane 301 with the 3D surface and segment the 2D image slices into groups corresponding to each tooth. In this particular embodiment, the plane is perpendicular to the z axis, and progresses along its normal along z direction through the extent of the 3D data. Initially, the intersection can be a number of small polygons as shown in the first row in Fig. 4, growing in size and merging as the plane moves downward. In some embodiments, central to recognizing the individual teeth is identifying the width and hence scale of the teeth. An exemplary tooth width is shown in Fig. 4. In some embodiments, the teeth width is along the x direction, y direction, or any other direction in the x-y plane. In some embodiments, the search for tooth width can be seeded with a reasonable average value or an input parameter as a reasonable estimate to start the search. Once the number of polygons is down to the expected number of teeth, a nominal center of each tooth can be located at the average of each group. This nominal center can then be used to segment the surface data higher from earlier (higher) slices along the z direction. With each slice lower, the average center position of each polyline, i.e., intersection of plane 301 and 3D surface, can remain close to the tooth nominal center until the surfaces start to join. The joining of the surfaces can be recognizable as a jump in center position as well as increase in lateral extents in the x-y plane. At this point, for instances, the sharpest cusp nominally midway between tooth nominal centers represents the transition between the adjacent teeth and the polylines can be segmented there. Continuing the process and the plane 301 can intersect the gumline, identifying the transition between tooth and gum may again involve searching the length of polyline for a distinct cusp (limiting search based on previously identified centers and nominal tooth width). Once the data in the raw 3D surface has completely segmented into teeth groups, the individual tooth surface models can be reconstructed. In some embodiments, missing data from the hidden portions of the scan are interpolated. Multidimensional radiographic (i.e. X-ray, CBCT, etc.) data can be used to confirm the interpolated data is correct. In some embodiments, processing the surface data comprises using interpolation for estimating interproximal contact, occluded interproximal contact, occluded subgingival contact, or any combination thereof.

Figs. 5A shows a 3D surface model of a tooth and Fig. 5B shows the crown preparation surface of the tooth in Fig. 5A.

### Methods and apparatus for automating the cutting of prosthetic margins

In some embodiments, the cutting of a tooth's prosthetic preparation margins by a dental operation system (e.g., ADD) provides the necessary clinically acceptable geometries that lead to the long term success of a dental crown. Such geometries can include but are not limited to: chamfer, knife edge, radial, radial with bevel, and square. In some embodiments, the tooth is automatically cut with a cutting bit, a cutting bur, laser ablation, a water jet, an air jet, an abrasive, or any combination thereof

In some embodiments, using diagnostic data, the clinical parameter, or both, for example, observation, surface mapping, radiographic data, ultrasound data, or any combination thereof, of the tooth, a tissue surrounding the tooth, or both the clinician can approve the final preparation material and shape before the operation begins, leaving the margins' shape to their discretion. The cutting bit can then be prescribed by a software to best match the intended shape of the margin and cut path, which can then be placed manually or automatically into a hand piece or an automated cutting head for cutting. In some embodiments, comprises a crown material.

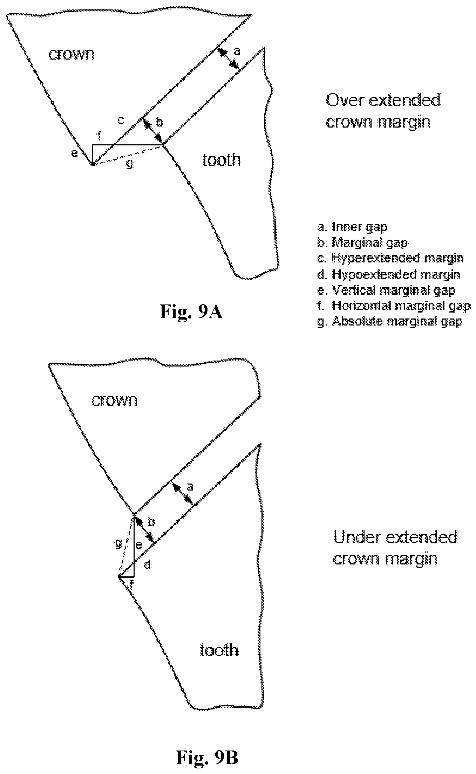

Referring to Figs. 9A-9B, in a particular embodiment, different elements associated with the crown margin are shown. In this embodiment, element a between the tooth and crown is an inner gap, element b is a marginal gap, c is a hyperextended margin, d is a hypoextended margin, e is a vertical marginal gap, f is a horizontal marginal gap, and g is an absolute marginal gap. In some embodiments, one or more of elements associated with the crown margin is used to determine the shape of the margin and/or the cut path.

### Methods and apparatus for automating the cutting of circumferential and occlusal tooth

### reductions

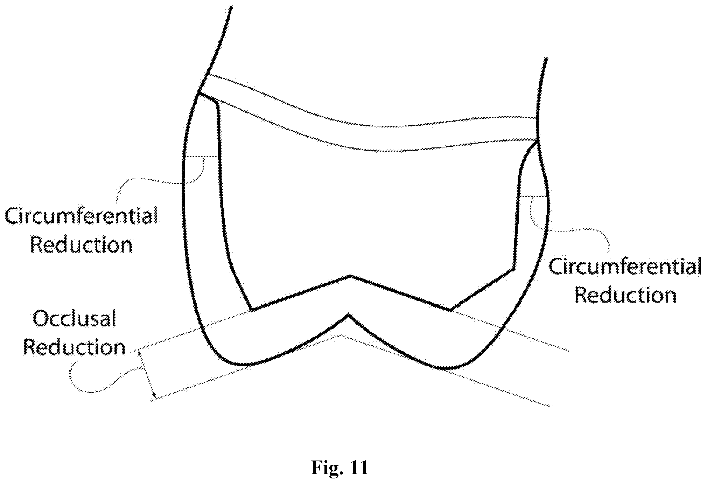

Provided herein in some embodiments, are methods and apparatus for automating the cutting of circumferential and occlusal tooth reductions. Figs. 11A and 11B show non-limiting illustrations of reductions for a molar or premolar tooth for installation prosthetic crown. In some embodiments, the cutting of a tooth beyond the prosthetic preparation margin includes both the definition of a circumferential or occlusal reduction with a material removal method. In some embodiments, the material removal method includes the determination of a consistent taper, and the taper being the angle of the straight-cut tooth preparation walls relative to the draw axis of the tooth for molars and premolars, and a consistent reduction for incisors. In some embodiments, the occlusal reduction is determined based on a shape of an opposing tooth, an adjacent tooth, the final external crown surface geometry, the cement gap distance, the FEA analysis of the crown or restoration combination, or any combination thereof to the tooth for installation of the crown.

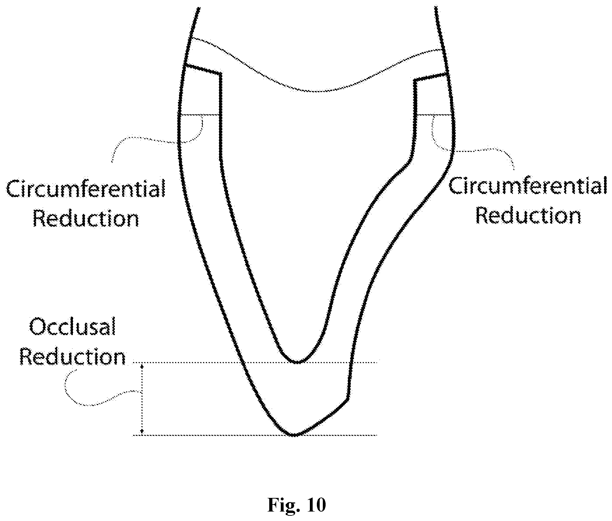

In some embodiments, assuming a complete crown, dimensions for reductions of the preparation are dependent on several things, including but not limited to: complete carious material removal, crown material, tooth position, tooth type, gums' health and position, and tooth health. Referring to Figs. 10-11, in particular embodiments, the circumferential reduction can be about 1mm and the occlusal reduction is about 1.5 mm.

In some embodiments, the system herein includes a laser generating source that generates a laser beam for cutting or drilling of the teeth. In some embodiments, the laser is generated at the distal end of the system, e.g., by incorporating a laser generating source in an ADD system. In some embodiments, the laser is generated and transmitted to the distal end of the system. In some embodiments, the clamp can be sized (e.g., recessed along z direction) so that it allows laser access to the teeth of the subject.

In some embodiments, the laser beam being generated has an operating wavelength in the range from about 0.1µm to about 50 µm. In some embodiments, the laser beam being generated has an operating wavelength in the range from about 1µm to about 50 µm. In some embodiments, the laser beam being generated has an operating wavelength in the range from about 5 µm to about 20 µm. In some embodiments, the laser beam being generated has an operating wavelength in the range from about 6 µm to about 15 µm. In some embodiments, the laser beam being generated operates at a plurality of wavelengths in the range from about 0.1µm to about 50 µm. In some embodiments, the laser beam being generated operates at a plurality of wavelengths in the range from about 1µm to about 50 µm. In some embodiments, the laser beam being generated operates at a plurality of wavelengths in the range from about 5 µm to about 50 µm. In some embodiments, the laser beam being generated operates at a plurality of wavelengths in the range from about 5µm to about 20 µm.

In some embodiments, the laser beam generated herein by the system is configured to provide different spot size suitable for different cutting or drilling applications. In some embodiments, the laser beam generated herein is switched on and off in a pulsed, periodic manner during cutting. In some embodiments, the duration and time between "on" pules may be controlled to optimize the cutting or drilling process. In some embodiments, the optical power of the laser beam generated herein may be controlled to optimize the cutting or drilling process. In some embodiments, the optic power of the laser beam generated herein may be varied from pulse to pulse in order to optimize the cutting or drilling process. In some embodiments, the optical power of the laser beam generated herein may be varied within a pulse in order to optimize the cutting or drilling process. In some embodiments, the laser-beam spot may be scanned within a localized region of the tooth, to optimize removal of tooth material at that region. In some embodiments, the laser-beam spot may be scanned within a localized region of the tooth, to optimize removal of gingiva at that region. In some embodiments, several or all of the spot size, spot scanning pattern, pulse repletion rate, pulse duration, and laser optical power may be controlled in concert to optimize the removal of tooth material. In some embodiments, several or all of the spot size, spot scanning pattern, pulse repletion rate, pulse duration, and laser optical power may be controlled in concert to optimize the removal of gingiva.

In some embodiments, the laser generating source is an neodymium-doped yttrium aluminum garnet laser (neodymium YAG, Nd:YAG). In some embodiments, the laser generating source emits light of approximate wavelength 0.946 µm. In some embodiments, the laser generating source emits light of approximate wavelength 1.12 µm. In some embodiments, the laser generating source emits light of approximate wavelength 1.32 µm. In some embodiments, the laser generating source emits light of approximate wavelength 1.44 µm.

In some embodiments, the laser generating source is an erbium-doped yttrium aluminum garnet laser (erbium YAG, Er:YAG). In some embodiments, the laser generating source emits light of approximate wavelength 2.94 µm.

In some embodiments, the laser generating source is a carbon-dioxide laser. In some embodiments, the laser generating source emits light of approximate wavelength 10 µm. In some embodiments, the laser generating source emits light or approximate wavelength 10.6 µm. In some embodiments, the laser generating source emits light or approximate wavelength 10.3 µm. In some embodiments, the laser generating source emits light or approximate wavelength 9.6 µm.

In some embodiments, the laser generating source emits light of approximate wavelength 9.3 µm, nearing the peak absorption of hydroxyapatite. In some embodiments, the gain medium of the laser generating source is a carbon-dioxide gas that includes an oxygen-18 isotope. In some embodiments, the laser herein includes an isotopic CO2 laser that vaporizes enamel and gingiva. In some embodiments, the laser is configured to allow fast and efficient cutting at any angle, with more speed, precision and less bleeding than traditional cutting or drilling methods. In some embodiments, the system comprising a laser beam for tooth or gingiva cutting or drilling does not require anesthesia of the subject.

In some embodiments, automation, e.g., through optical tracking methods, is required to judge how much material has been removed using the laser cutting methods and the laser generating system herein.

In some embodiments the tooth preparation surface and prosthetic surface may be generated together. In some embodiments, the prosthetic external surface is generated as a function of the volumetric boundary for the prosthetic. In some embodiments, the prosthetic external surface includes the proximal contacts of adjacent teeth including opposing the occlusion of the teeth and the margin of the prosthetic. In some embodiments, the internal surface of the prosthetic is determined based on one or more of the external surface and the material thickness, a result of a required removal of carious or otherwise damaged portions of the tooth, and another constraint associated with the standard of care for preparations. In some embodiments, the internal preparation surface of the prosthetic matches the preparation geometry offset by a cement thickness. In some embodiments, the internal preparation surface of the prosthetic matches the preparation geometry offset by a cement thickness, with a coincident marginal line between the preparation and crown margins. In some embodiments, once the surfaces are generated, a software module performs Finite Element Analysis (FEA) to iterate the prosthetic internal surface, the preparation surface, or both to optimize the stress and/or strain within the tooth and/or prosthetic. In some embodiments, the FEA further enables optimization of crown and tooth geometry and thickness to reduce risk associated with high forces (i.e. fracture or chipping).

### Digital processing device

In some embodiments, the systems, and methods described herein include a digital processing device, or use of the same. In further embodiments, the digital processing device includes one or more hardware central processing units (CPUs) or general purpose graphics processing units (GPGPUs) that carry out the device's functions. In still further embodiments, the digital processing device further comprises an operating system configured to perform executable instructions. In some embodiments, the digital processing device is optionally connected to a computer network. In further embodiments, the digital processing device is optionally connected to the Internet such that it accesses the World Wide Web. In still further embodiments, the digital processing device is optionally connected to a cloud computing infrastructure. In other embodiments, the digital processing device is optionally connected to an intranet. In other embodiments, the digital processing device is optionally connected to a data storage device.

In accordance with the description herein, suitable digital processing devices include, by way of non-limiting examples, server computers, desktop computers, laptop computers, notebook computers, sub-notebook computers, netbook computers, netpad computers, set-top computers, media streaming devices, handheld computers, Internet appliances, mobile smartphones, tablet computers, and personal digital assistants.

In some embodiments, the digital processing device includes an operating system configured to perform executable instructions. The operating system is, for example, software, including programs and data, which manages the device's hardware and provides services for execution of applications.

In some embodiments, the device includes a storage and/or memory device. The storage and/or memory device is one or more physical apparatuses used to store data or programs on a temporary or permanent basis.

In some embodiments, the digital processing device includes a display to send visual information to a user.

In some embodiments, the digital processing device includes an input device to receive information from a user.

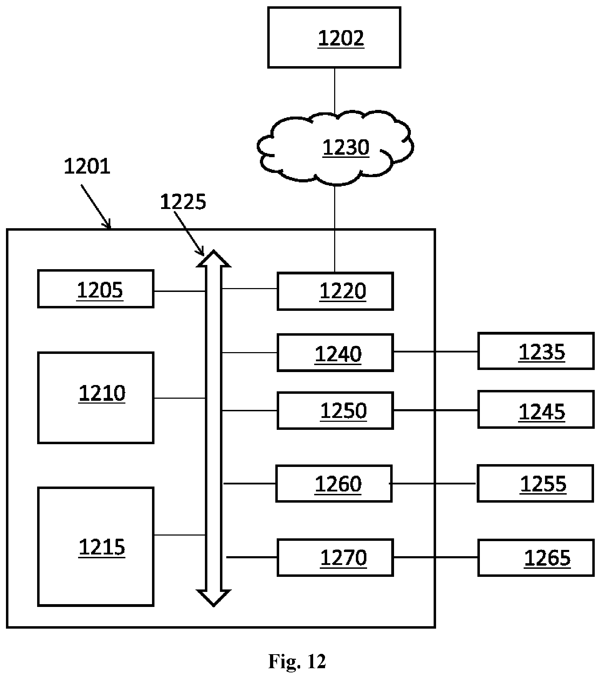

Referring to Fig. 12, in a particular embodiment, an exemplary digital processing device 1201 is programmed or otherwise configured to control surface data communication, surface data processing, generation of one or more parameters of the tooth, generation of the crown preparation surface, the crown cavity surface, and margin(s). In this embodiment, the digital processing device 1201 includes a central processing unit (CPU, also "processor" and "computer processor" herein) 1205, which can be a single core or multi core processor, or a plurality of processors for parallel processing. The digital processing device 1201 also includes memory or memory location 1210 (e.g., random-access memory, read-only memory, flash memory), electronic storage unit 1215 (e.g., hard disk), communication interface 1220 (e.g., network adapter, network interface) for communicating with one or more other systems, and peripheral devices, such as cache, other memory, data storage and/or electronic display adapters. The peripheral devices can include storage device(s) or storage medium 1265 which communicate with the rest of the device via a storage interface 1270. The memory 1210, storage unit 1215, interface 1220 and peripheral devices are in communication with the CPU 1205 through a communication bus 1225, such as a motherboard. The storage unit 1215 can be a data storage unit (or data repository) for storing data. The digital processing device 1201 can be operatively coupled to a computer network ("network") 1230 with the aid of the communication interface 1220. The network 1230 can be the Internet, an internet and/or extranet, or an intranet and/or extranet that is in communication with the Internet. The network 1230 in some cases is a telecommunication and/or data network. The network 1230 can include one or more computer servers, which can enable distributed computing, such as cloud computing. The network 1230, in some cases with the aid of the device 1201, can implement a peer-to-peer network, which can enable devices coupled to the device 1201 to behave as a client or a server.

Continuing to refer to Fig. 12, the digital processing device 1201 includes input device(s) 12125 to receive information from a user, the input device(s) in communication with other elements of the device via an input interface 1250. The digital processing device 1201 can include output device(s) 1255 that communicates to other elements of the device via an output interface 1260.

Continuing to refer to Fig. 12, the memory 1210 can include various components (e.g., machine readable media) including, but not limited to, a random-access memory component (e.g., RAM) (e.g., a static RAM "SRAM", a dynamic RAM "DRAM, etc.), or a read-only component (e.g., ROM). The memory 1210 can also include a basic input/output system (BIOS), including basic routines that help to transfer information between elements within the digital processing device, such as during device start-up, can be stored in the memory 1210.

Continuing to refer to Fig. 12, the CPU 1205 can execute a sequence of machine-readable instructions, which can be embodied in a program or software. The instructions can be stored in a memory location, such as the memory 1210. The instructions can be directed to the CPU 1205, which can subsequently program or otherwise configure the CPU 1205 to implement methods of the present disclosure. Examples of operations performed by the CPU 1205 can include fetch, decode, execute, and write back. The CPU 1205 can be part of a circuit, such as an integrated circuit. One or more other components of the device 1201 can be included in the circuit. In some cases, the circuit is an application specific integrated circuit (ASIC) or a field programmable gate array (FPGA).

Continuing to refer to Fig. 12, the storage unit 1215 can store files, such as drivers, libraries and saved programs. The storage unit 1215 can store user data, e.g., user preferences and user programs. The digital processing device 1201 in some cases can include one or more additional data storage units that are external, such as located on a remote server that is in communication through an intranet or the Internet. The storage unit 1215 can also be used to store operating system, application programs, and the like. Optionally, storage unit 1215 can be removably interfaced with the digital processing device (e.g., via an external port connector (not shown)) and/or via a storage unit interface. Software may reside, completely or partially, within a computer-readable storage medium within or outside of the storage unit 1215. In another example, software may reside, completely or partially, within processor(s) 1205.

Continuing to refer to Fig. 12, the digital processing device 1201 can communicate with one or more remote computer systems 1202 through the network 1230. For instance, the device 1201 can communicate with a remote computer system of a user. Examples of remote computer systems include personal computers (e.g., portable PC), slate or tablet PCs (e.g., Apple^{®} iPad, Samsung^{®} Galaxy Tab), telephones, Smart phones (e.g., Apple^{®} iPhone, Android-enabled device, Blackberry^{®}), or personal digital assistants. In some embodiments, the remote computer system is configured for image and signal processing of images acquired using the image systems herein. In some embodiments, the imaging systems herein allows partitioning of image and signal processing between a processor in the imaging head (e.g. based on a MCU, DSP or FPGA) and a remote computer system, i.e., a back-end server.

Continuing to refer to Fig. 12, information and data can be displayed to a user through a display 1235. The display is connected to the bus 1225 via an interface 12120, and transport of data between the display other elements of the device 1201 can be controlled via the interface 12120.

Methods as described herein can be implemented by way of machine (e.g., computer processor) executable code stored on an electronic storage location of the digital processing device 1201, such as, for example, on the memory 1210 or electronic storage unit 1215. The machine executable or machine-readable code can be provided in the form of software. During use, the code can be executed by the processor 1205. In some cases, the code can be retrieved from the storage unit 1215 and stored on the memory 1210 for ready access by the processor 1205. In some situations, the electronic storage unit 1215 can be precluded, and machineexecutable instructions are stored on memory 1210.

### Radiographic referencing and density data mapping

Provided herein are methods and system for organizing dental diagnostic data by registering a 2D/3D radiograph to a 3D surface scan to inform intraoral automated cutting.

In some embodiments, the methods comprise combining and registering a 2D bitewing radiograph or a 3D CT radiograph to a 3D surface scan. In some embodiments, the 2D radiograph provides cross-sectional detail of the tooth , wherein the cross section is a targeted registration point formed when combining with the surface data. In some embodiments, similar to the 2D radiograph, the 3D CT has a 3-axis datum and registration point that provides reference to the surface data. In some embodiments, certain landmarks are leveraged for registration. In some embodiments, the landmark comprises a crestal bone, a periodontal ligament space, a pulp chamber, gingival margin, biological width, or any combination thereof . This results in a detailed representation of the tooth throughout its volume, for improved diagnostics during presurgery planning. In some embodiments, an interface between an internal structure of the pulp and dentin provides margin planning collateral. In some embodiments, a visible fiducial, radiographic fiducial, or both is employed in the assessments, in the case that landmarks do not provide adequate registration for the coupling of the two image profiles.

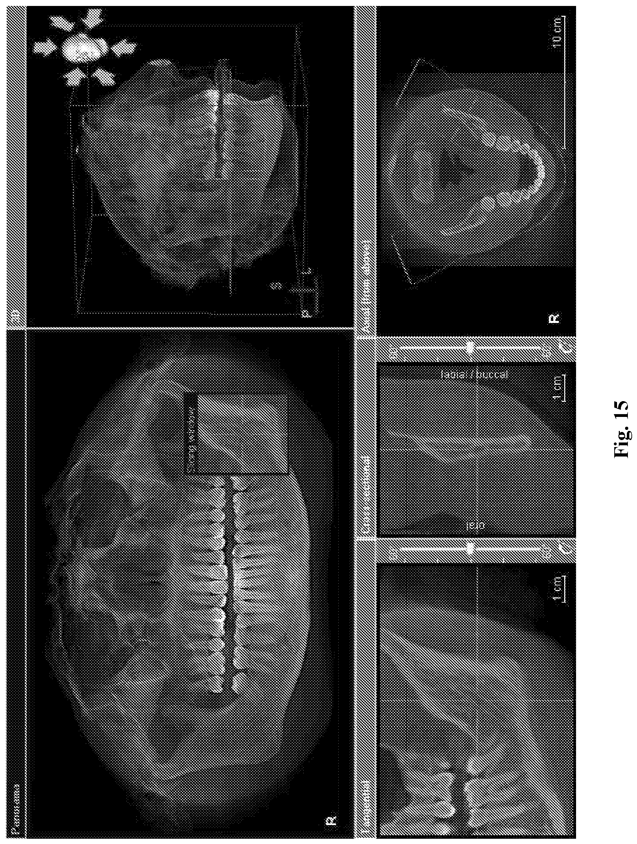

In some embodiments, registration of the radiological and surface data is performed manually by a trained operator. Preferably, in some embodiments, registration of the radiological and surface data is performed automatically through a best fit of common features between the two data sets. In one example, , per FIG. 15A, a normal projection of the 3D surface of the subject tooth onto the plane 3 of the 2D bitewing radiograph provides the area of the cross section 4 per FIG. 15B. In some embodiments, the boundary of the cross section 5 corresponds to a boundary of the shadow of the subject tooth in the radiograph. In some embodiments, the upper portion of the boundary 5 is the most reliable data portion of the 3D surface, as it is derived directly from the raw scan data. Therefore, much of the root and adjacent surfaces will be extrapolated or derived from radiology.

In some embodiments, the registration process between the surface derived boundary and upper boundary of the radiograph uses conventional image correlation techniques of the boundary polylines. Registration landmarks (either anatomical or pre-applied fiducials), in some embodiments, are used as an alternative to the 3D surface projection or to assist in the process. If the actual plane of the 2D radiograph is unknown, in some embodiments, it may be necessary to generate a range of projection angles and select the best fit.

### Apparatus of combining real-time density calculations

Also provided herein are methods and apparatus of combining real-time density calculations using motor torque and/or shaft levering mapped to 3D diagnostic data, the clinical parameter, or both, along a surgical toolpath to inform intraoral automated cutting

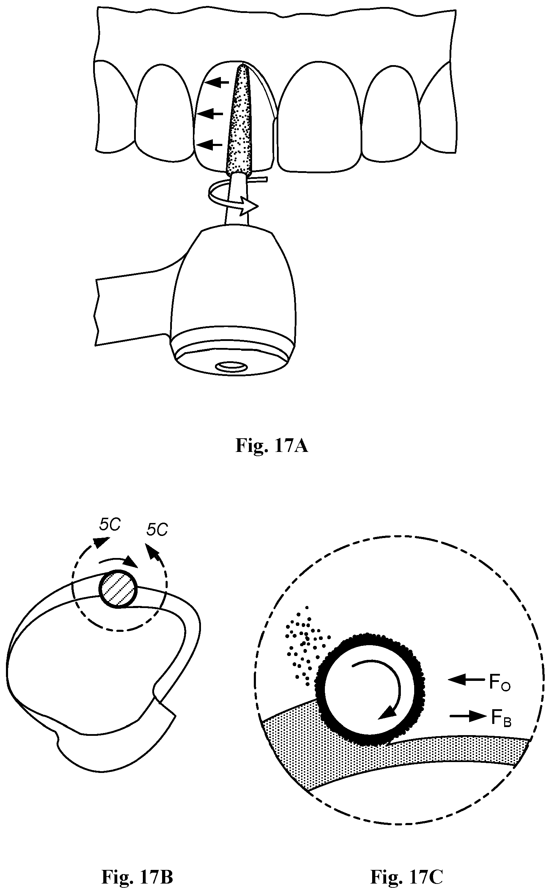

The present disclosure describes the combination of density calculations through tracking motor torque with a predetermined 3D data set of a tooth topography. In some embodiments, measured against an expected nominal value and with the line of contact/depth of cut known, the type of tissue being cut at a given time can be approximated. Thus, various tissues and amalgam materials can be mapped to tooth regions. In some embodiments, a loss of torque during cutting indicates a lack of contact with the tooth, providing a safety shutoff method. In some embodiments, a method of shaft leverage is employed to measure force perpendicular to the axis of the cutter. Figs 17A-17C shows an exemplary diagram of burr forces.

Given known motor speed, depth and width of cut, in some embodiments, the force required (as measured by motor power) varies with the density of the material being cut. The expected material density can be derived from the 3D model and relevant cross section of the subject tooth. Correlation between the actual and expected torque provides confidence the cutting is proceeding as planned. In some embodiments, a variation outside of the measurement/modeling tolerances is an indication of an error condition, providing a mechanism to safely shutoff or alert a device user/clinician.

Motor power is easily measured in the case of an electric drill as is currently envisaged, due to the drivetrain from the end effector providing motor torque feedback that can be related to cutting resistance due to material hardness/toughness. Pneumatic drills' speed can be monitored over time allowing the corresponding torque difference to be derived and related to the varying cutting force at the material's surface.

Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. In some embodiments, the computer memory comprises machine executable code that, upon execution by the one or more computer processors, implements any of the methods above or elsewhere herein.

### Non-transitory computer readable storage medium

In some embodiments, the platforms, systems, media, and methods disclosed herein include one or more non-transitory computer readable storage media encoded with a program including instructions executable by the operating system of an optionally networked digital processing device. In further embodiments, a computer readable storage medium is a tangible component of a digital processing device. In still further embodiments, a computer readable storage medium is optionally removable from a digital processing device. In some embodiments, a computer readable storage medium includes, by way of non-limiting examples, CD-ROMs, DVDs, flash memory devices, solid state memory, magnetic disk drives, magnetic tape drives, optical disk drives, cloud computing systems and services, and the like. In some cases, the program and instructions are permanently, substantially permanently, semi-permanently, or nontransitorily encoded on the media.

### Computer program

In some embodiments, the platforms, systems, media, and methods disclosed herein include at least one computer program, or use of the same. A computer program includes a sequence of instructions, executable in the digital processing device's CPU, written to perform a specified task. Computer readable instructions may be implemented as program modules, such as functions, objects, Application Programming Interfaces (APIs), data structures, and the like, that perform particular tasks or implement particular abstract data types. In light of the disclosure provided herein, those of skill in the art will recognize that a computer program may be written in various versions of various languages.

The functionality of the computer readable instructions may be combined or distributed as desired in various environments. In some embodiments, a computer program comprises one sequence of instructions. In some embodiments, a computer program comprises a plurality of sequences of instructions. In some embodiments, a computer program is provided from one location. In other embodiments, a computer program is provided from a plurality of locations. In various embodiments, a computer program includes one or more software modules. In various embodiments, a computer program includes, in part or in whole, one or more web applications, one or more mobile applications, one or more standalone applications, one or more web browser plug-ins, extensions, add-ins, or add-ons, or combinations thereof.

### Web application

In some embodiments, a computer program includes a web application. In light of the disclosure provided herein, those of skill in the art will recognize that a web application, in various embodiments, utilizes one or more software frameworks and one or more database systems. In some embodiments, a web application is created upon a software framework such as Microsoft^{®} .NET or Ruby on Rails (RoR). In some embodiments, a web application utilizes one or more database systems including, by way of non-limiting examples, relational, non-relational, object oriented, associative, and XML database systems. In further embodiments, suitable relational database systems include, by way of non-limiting examples, Microsoft^{®} SQL Server, mySQL^{™}, and Oracle^{®}.

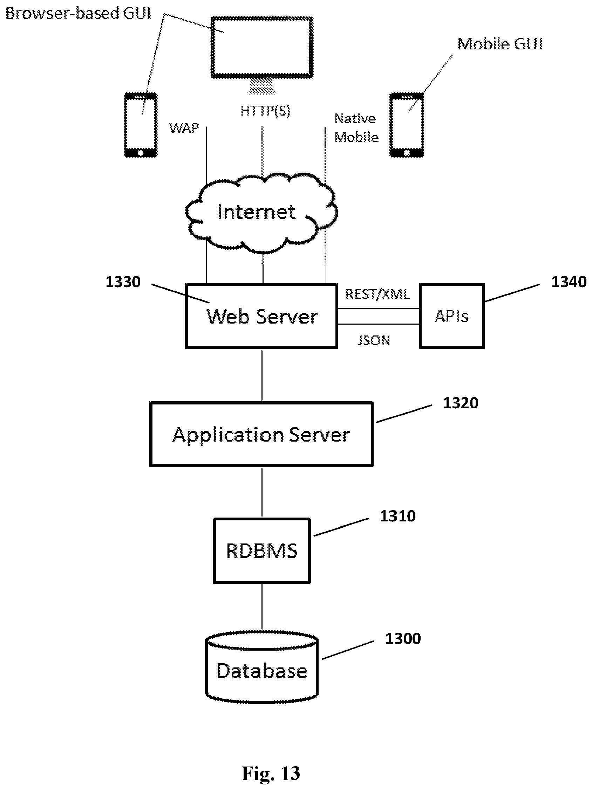

Referring to Fig. 13, in a particular embodiment, an application provision system comprises one or more databases 1300 accessed by a relational database management system (RDBMS) 1310. Suitable RDBMSs include Firebird, MySQL, PostgreSQL, SQLite, Oracle Database, Microsoft SQL Server, IBM DB2, IBM Informix, SAP Sybase, SAP Sybase, Teradata, and the like. In this embodiment, the application provision system further comprises one or more application severs 1320 (such as Java servers, .NET servers, PHP servers, and the like) and one or more web servers 1330 (such as Apache, IIS, GWS and the like). The web server(s) optionally expose one or more web services via app application programming interfaces (APIs) 1340. Via a network, such as the Internet, the system provides browser-based and/or mobile native user interfaces.

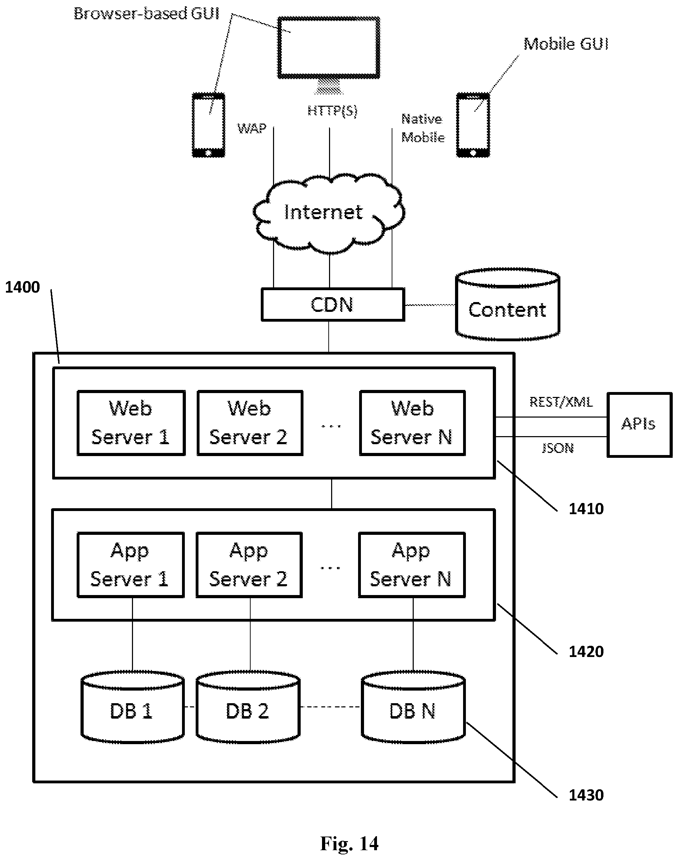

Referring to Fig. 14, in a particular embodiment, an application provision system alternatively has a distributed, cloud-based architecture 1400 and comprises elastically load balanced, auto-scaling web server resources 1410 and application server resources 1420 as well synchronously replicated databases 1430.

### Software modules

In some embodiments, the platforms, systems, media, and methods disclosed herein include software, server, and/or database modules, or use of the same. In view of the disclosure provided herein, software modules are created by techniques known to those of skill in the art using machines, software, and languages known to the art. The software modules disclosed herein are implemented in a multitude of ways. In various embodiments, a software module comprises a file, a section of code, a programming object, a programming structure, or combinations thereof. In further various embodiments, a software module comprises a plurality of files, a plurality of sections of code, a plurality of programming objects, a plurality of programming structures, or combinations thereof. In various embodiments, the one or more software modules comprise, by way of non-limiting examples, a web application, a mobile application, and a standalone application. In some embodiments, software modules are in one computer program or application. In other embodiments, software modules are in more than one computer program or application. In some embodiments, software modules are hosted on one machine. In other embodiments, software modules are hosted on more than one machine. In further embodiments, software modules are hosted on cloud computing platforms. In some embodiments, software modules are hosted on one or more machines in one location. In other embodiments, software modules are hosted on one or more machines in more than one location.

### Databases

In some embodiments, the platforms, systems, media, and methods disclosed herein include one or more databases, or use of the same. In view of the disclosure provided herein, those of skill in the art will recognize that many databases are suitable for storage and retrieval of surface data of a tooth, crown preparation surface, crown cavity surface, margin, toolpaths, etc. In various embodiments, suitable databases include, by way of non-limiting examples, relational databases, non-relational databases, object oriented databases, object databases, entityrelationship model databases, associative databases, and XML databases. Further non-limiting examples include SQL, PostgreSQL, MySQL, Oracle, DB2, and Sybase. In some embodiments, a database is internet-based. In further embodiments, a database is web-based. In still further embodiments, a database is cloud computing-based. In other embodiments, a database is based on one or more local computer storage devices.

Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

### Machine Learning

In some embodiments, at least a portion of the prosthetic and preparation surfaces are generated by a machine learning algorithm. In some embodiments, the machine learning algorithm comprises a neural network.

In some embodiments, the software can generate incomplete or missing scanned surfaces of the teeth by interpreting one or more missing surface patches. In some embodiments, the one or more missing surface patches comprise an occluded and/or interproximal region between teeth or a subgingival tooth surface. In some embodiments, the one or more missing surface patches are patched by interpolation of the expected surface from existing scanned geometries, a machine learning algorithm, a neural network, or any combination thereof. In some embodiments, the one or more missing surface patches are patched by normalized tooth geometries from marked samples. In some embodiments, the one or more missing surface patches by combining conventional dental scanning with optical coherence tomography of occluded or hidden (subgingival) surfaces.

Examples of machine learning algorithms comprise a feedforward neural network, a recurrent neural network, a convolutional neural network, a generative adversarial networks (GANS) including but not limited to voxels and point clouds, visual object networks, or any combination thereof. In some embodiments, the GANS comprises a voxel, a point cloud, or both. In some embodiments, Optical Coherence Tomography (OCT) is used to scan critical subgingival surfaces of the tooth. The resulting OCT surface scan can be stitched together with the conventional surface scan to form a master surface that includes all critical surfaces for the procedure.

In some embodiments, machine learning algorithms are utilized to aid in determining the one or more missing surface patches. In some embodiments, the machine learning algorithms herein determine the one or more missing surface patches using labels including but not limited to human annotated labels and semi-supervised labels. The human annotated labels can be provided by a hand-crafted heuristic. The semi-supervised labels can be determined using a clustering technique to find properties similar to those flagged by previous human annotated labels and previous semi-supervised labels. The semi-supervised labels can employ a XGBoost, a neural network, or both.

In some embodiments, the machine learning algorithms herein determine the one or more missing surface patches using a distant supervision method. The distant supervision method can create a large training set seeded by a small hand-annotated training set. The distant supervision method can comprise positive-unlabeled learning with the training set as the 'positive' class. The distant supervision method can employ a logistic regression model, a recurrent neural network, or both. The recurrent neural network can be advantageous for Natural Language Processing (NLP) machine learning.

Examples of machine learning algorithms can include a support vector machine (SVM), a naive Bayes classification, a random forest, a neural network, deep learning, or other supervised learning algorithm or unsupervised learning algorithm for classification and regression. The machine learning algorithms can be trained using one or more training datasets.

In some embodiments, the machine learning algorithm utilizes regression modeling, wherein relationships between predictor variables and dependent variables are determined and weighted.

In some embodiments, a machine learning algorithm is used to select catalogue images and recommend project scope. A non-limiting example of a multi-variate linear regression model algorithm is seen below: probability = A₀ + A₁(X₁) + A₂(X₂) + A₃(X₃) + A₄(X₄) + A₅(X₅) + A₆(X₆) + A₇(X₇)...wherein Aᵢ (A₁, A₂, A₃, A₄, A₅, A₆, A₇, ...) are "weights" or coefficients found during the regression modeling; and Xᵢ (X₁, X₂, X₃, X₄, X₅, X₆, X₇, ...) are data collected from the User. Any number of Aᵢ and Xᵢ variable can be included in the model. In some embodiments, the programming language "R" is used to run the model.

In some embodiments, training comprises multiple steps. At least one of the first step, the second step, and the third step can repeat one or more times continuously or at set intervals.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

While various embodiments of the invention have been shown and described herein, it will be obvious to those skilled in the art that such embodiments are provided by way of example only. Numerous variations, changes, and substitutions may occur to those skilled in the art without departing from the invention as defined by the appended claims. It should be understood that various alternatives to the embodiments of the invention described herein may be employed.

## Claims

1. A computer-implemented method for generating a preparation surface of a tooth of a subject, the method comprising computer implemented steps of:
a) receiving radiological surface data of the tooth of the subject,
b) determining one or more parameters of the tooth of the subject by analyzing the surface data, and
c) generating the preparation surface using the one or more parameters,
wherein the preparation surface of the tooth comprises a three-dimensional model of the surface of the tooth and a cut region,
wherein the surface data comprises data with multiple orientations or data with full 3D details, and wherein the method further comprises computer implemented steps of:
determining an extent of tooth decay by analyzing the surface data, and
determining at least one of a top surface and an edge of the preparation surface, based on said extent of tooth decay.

2. The method of claim 1, further comprising, subsequent to c, automatically generating a crown cavity surface by adding a pre-determined gap space to the crown preparation surface.

3. The method of claim 2, wherein the pre-determined gap space is based on a manufacturing tolerance of a crown cavity, a machining tolerance of the crown preparation surface, a desired marginal gap, or any combination thereof.

4. The method of claim 2 or claim 3, further comprising: combining the crown cavity surface with an outer crown surface to generate a 3D volume of a manufactured crown, identifying one or more areas having a thickness less than a minimum thickness, and growing the crown cavity surface inwards in said one or more areas, with the preparation surface moving inward accordingly in said areas to retain said gap space.

5. The method of any preceding claim, wherein the surface data comprises computed tomography data.

6. The method of claim 5, wherein the surface data comprises cone beam computed tomography data.

7. The method of any preceding claim, further comprising, subsequent to c), transmitting the preparation surface to a system configured for toolpath generation, machining of the tooth, or both.

8. The method of any preceding claim, further comprising, subsequent to c), transmitting the preparation surface to a system configured for a dental procedure.

9. The method of claim 8, wherein the system configured for the dental procedure is an automated dental drill system configured for tooth cutting or tooth drilling.

10. The method of claim 9, wherein the automated dental drill system comprises a laser generating source.

11. A system comprising one or more computer processors (1205) and computer memory (1210) coupled thereto, the computer memory comprising machine executable code that, upon execution by the one or more computer processors, implements the method of any one of claims 1-10.

12. A computer program product comprising instructions that, upon execution by one or more computer processors, implement the method of any one of claims 1-10.

## Patentansprüche

1. Computerimplementiertes Verfahren zum Erzeugen einer Präparationsoberfläche eines Zahns eines Subjekts, wobei das Verfahren die folgenden computerimplementierten Schritte umfasst:
a) Empfangen von radiologischen Oberflächendaten des Zahns des Subjekts,
b) Bestimmen eines oder mehrerer Parameter des Zahns des Subjekts durch Analysieren der Oberflächendaten, und
c) Erzeugen der Präparationsoberfläche unter Verwendung des einen oder der mehreren Parameter,
wobei die Präparationsoberfläche des Zahns ein dreidimensionales Modell der Oberfläche des Zahns und eine Schneidregion umfasst,
wobei die Oberflächendaten Daten mit mehreren Orientierungen oder Daten mit vollständigen 3D-Details umfassen, und wobei das Verfahren ferner die folgenden computerimplementierten Schritte umfasst:
Bestimmen eines Ausmaßes des Zahnverfalls durch Analysieren der Oberflächendaten, und
Bestimmen von mindestens einer von einer oberen Oberfläche und einer Kante der Präparationsoberfläche, basierend auf dem Ausmaß des Zahnverfalls.

2. Verfahren nach Anspruch 1, ferner umfassend, nach c, automatisches Erzeugen einer Kronenhohlraumoberfläche durch Hinzufügen eines vorbestimmten Zwischenraumraums zu der Kronenpräparationsoberfläche.

3. Verfahren nach Anspruch 2, wobei der vorbestimmte Zwischenraumraum auf einer Fertigungstoleranz eines Kronenhohlraums, einer Bearbeitungstoleranz der Kronenpräparationsoberfläche, einem gewünschten Randzwischenraum oder einer beliebigen Kombination davon basiert.

4. Verfahren nach Anspruch 2 oder Anspruch 3, ferner umfassend: Kombinieren der Kronenhohlraumoberfläche mit einer äußeren Kronenoberfläche, um ein 3D-Volumen einer gefertigten Krone zu erzeugen, Identifizieren eines oder mehrerer Bereiche mit einer Dicke, die geringer als eine Mindestdicke ist, und Wachsen der Kronenhohlraumoberfläche nach innen in dem einen oder den mehreren Bereichen, wobei sich die Präparationsoberfläche entsprechend in den Bereichen nach innen bewegt, um den Zwischenraumraum beizubehalten.

5. Verfahren nach einem der vorhergehenden Ansprüche, wobei die Oberflächendaten Computertomographiedaten umfassen.

6. Verfahren nach Anspruch 5, wobei die Oberflächendaten Kegelstrahl-Computertomographiedaten umfassen.

7. Verfahren nach einem der vorhergehenden Ansprüche, ferner umfassend, nach c), Übertragen der Präparationsoberfläche an ein System, das zur Werkzeugbahnerzeugung, Bearbeitung des Zahns oder beidem konfiguriert ist.

8. Verfahren nach einem der vorhergehenden Ansprüche, ferner umfassend, nach c), Übertragen der Präparationsoberfläche an ein System, das für ein zahnärztliches Verfahren konfiguriert ist.

9. Verfahren nach Anspruch 8, wobei das System, das für das zahnärztliche Verfahren konfiguriert ist, ein automatisiertes zahnärztliches Bohrsystem ist, das zum Zahnschneiden oder Zahnbohren konfiguriert ist.

10. Verfahren nach Anspruch 9, wobei das automatisierte zahnärztliche Bohrsystem eine Lasererzeugungsquelle umfasst.

11. System, das einen oder mehrere Computerprozessoren (1205) und einen damit gekoppelten Computerspeicher (1210) umfasst, wobei der Computerspeicher maschinenausführbaren Code umfasst, der bei Ausführung durch den einen oder die mehreren Computerprozessoren das Verfahren nach einem der Ansprüche 1-10 implementiert.

12. Computerprogrammprodukt, das Anweisungen umfasst, die bei Ausführung durch einen oder mehrere Computerprozessoren das Verfahren nach einem der Ansprüche 1-10 implementieren.

## Revendications

1. Procédé mis en oeuvre par ordinateur pour générer une surface de préparation d'une dent d'un sujet, le procédé comprenant les étapes mises en oeuvre par ordinateur consistant à :
a) recevoir des données de surface radiologique de la dent du sujet,
b) déterminer un ou plusieurs paramètres de la dent du sujet en analysant les données de surface, et
c) générer la surface de préparation en utilisant les un ou plusieurs paramètres,
dans lequel la surface de préparation de la dent comprend un modèle tridimensionnel de la surface de la dent et une région de coupe,
dans lequel les données de surface comprennent des données avec de multiples orientations ou des données avec des détails 3D complets, et dans lequel le procédé comprend en outre les étapes mises en oeuvre par ordinateur consistant à :
déterminer une étendue de dégradation dentaire en analysant les données de surface, et
déterminer au moins l'un d'une surface supérieure et d'un bord de la surface de préparation, sur la base de ladite étendue de dégradation dentaire.

2. Procédé selon la revendication 1, comprenant en outre, après l'étape c, la génération automatique d'une surface de cavité de couronne en ajoutant un espace d'espacement prédéterminé à la surface de préparation de couronne.

3. Procédé selon la revendication 2, dans lequel l'espace d'espacement prédéterminé est basé sur une tolérance de fabrication d'une cavité de couronne, une tolérance d'usinage de la surface de préparation de couronne, un espace marginal souhaité, ou toute combinaison de ceux-ci.

4. Procédé selon la revendication 2 ou la revendication 3, comprenant en outre : la combinaison de la surface de cavité de couronne avec une surface de couronne externe pour générer un volume 3D d'une couronne fabriquée, l'identification d'une ou plusieurs zones ayant une épaisseur inférieure à une épaisseur minimale, et la croissance de la surface de cavité de couronne vers l'intérieur dans lesdites une ou plusieurs zones, avec la surface de préparation se déplaçant vers l'intérieur en conséquence dans lesdites zones pour conserver ledit espace d'espacement.

5. Procédé selon l'une quelconque des revendications précédentes, dans lequel les données de surface comprennent des données de tomodensitométrie.

6. Procédé selon la revendication 5, dans lequel les données de surface comprennent des données de tomodensitométrie à faisceau conique.

7. Procédé selon l'une quelconque des revendications précédentes, comprenant en outre, après l'étape c), la transmission de la surface de préparation à un système configuré pour la génération de trajectoire d'outil, l'usinage de la dent, ou les deux.

8. Procédé selon l'une quelconque des revendications précédentes, comprenant en outre, après l'étape c), la transmission de la surface de préparation à un système configuré pour une procédure dentaire.

9. Procédé selon la revendication 8, dans lequel le système configuré pour la procédure dentaire est un système de fraisage dentaire automatisé configuré pour la coupe des dents ou le forage des dents.

10. Procédé selon la revendication 9, dans lequel le système de fraisage dentaire automatisé comprend une source de génération de laser.

11. Système comprenant un ou plusieurs processeurs informatiques (1205) et une mémoire informatique (1210) couplée à ceux-ci, la mémoire informatique comprenant un code exécutable par machine qui, lors de l'exécution par les un ou plusieurs processeurs informatiques, met en oeuvre le procédé selon l'une quelconque des revendications 1 à 10.

12. Produit de programme informatique comprenant des instructions qui, lors de l'exécution par un ou plusieurs processeurs informatiques, mettent en oeuvre le procédé selon l'une quelconque des revendications 1 à 10.
